# EUROPEAN PATENT APPLICATION

(11) **EP 2 039 385 A1**
(43) Date of publication of application: **25.03.2009**
(21) Application number: 07253700.4
(22) Date of filing: 18.09.2007
(51) Int. Cl.: A61M 5/50, A61M 5/315, A61M 5/32

(54) **Disposable syringe guarded in a preuse position (I)**

(71) Applicant: Shue, Ming-Jeng, Hsi Dist, Taichung City (TW); Shue, Phillip, Hsi District, Taichung City (TW); Huang, Deborah, Chung Dist Taichung City (TW)
(72) Inventor: Shue, Ming-Jeng, Hsi Dist, Taichung City (TW); Shue, Phillip, Hsi District, Taichung City (TW); Huang, Deborah, Chung Dist Taichung City (TW)
(74) Representative: Mackenzie, Andrew Bryan

(57) **Abstract**

A disposable syringe includes a barrel (1), a needle assembly (2), a plunger (3), and a spacer (4). A tubular catch (134) extends rearwardly of the barrel (1) to terminate at a peripheral edge (139). The needle assembly (2) includes a needle cannula (22) and a needle seat (21) for securing the needle cannula (22). The plunger (3) is movable in the barrel (1) to a retraction initiating position, where a front open plunger end (37) is coupled with the needle seat (21) for withdrawal of the needle seat (21) and the needle cannula (22) into the plunger (3). An endcap (33) is disposed to cover a rear open plunger end (38), and has a ring wall (332) fittable in the catch (134). The spacer (4) has front and rear abutment surfaces (411,412)that detachably and respectively abut against the peripheral edge (139) and a rim (333) of the ring wall (332) in a pre-use position to thereby prevent movement of the plunger (3) to the retraction initiating position.

## Description

This invention relates to a disposable syringe, more particularly to a disposable syringe which has a spacer to guard a plunger against undesired movement to a needle retraction initiating position so as to guard the syringe in a preuse position.

Conventional medical devices or syringes for injection medicine, drawing blood samples, etc., have to be disposed of safely after injection in order to avoid accidental needle pricks or undesirable contamination. Although a tip protector is provided to be sleeved on the syringe after use to ensure that the needle is covered, the user is exposed to the risk of being pricked by the needle when sleeving the tip protector on the syringe. Therefore, there are available medical devices or syringes with a retractable needle that is retractable into a barrel or a plunger after the injection operation is completed, such as those disclosed in U.S. Patent Nos. 6,743,199 and 6,921,386 issued to the applicants, and U.S. Patent Application Nos. 10/918,020 and 11/320, 927 filed by the applicants. However, further improvements are desirable to prevent undesired withdrawal of the needle caused by inadvertent pressing of the plunger in a pre-use position, to ensure successful withdrawal of the needle, and to have a slenderized construction for small volume injection.

One object of the present invention is to provide a disposable syringe which can be guarded in a preuse position to prevent undesired withdrawal of a needle caused by inadvertent pressing of the plunger in the preuse position.

Another object of the present invention is to provide a disposable syringe which can ensure successful withdrawal of a needle.

Still another object of the present invention is to provide a disposable syringe which has a slenderized construction for small volume injection.

According to this invention, the disposable syringe includes a barrel, a needle assembly, a plunger, and a spacer. The barrel has front and rear open barrel ends opposite to each other along an axis, and a surrounding barrel wall which defines a passage. A pair of lugs extend radially from the surrounding barrel wall and adjacent to the rear open barrel end. A tubular catch extends from the lugs along the axis and away from the surrounding barrel wall to terminate at a peripheral edge, and has outer and inner surrounding surfaces. The inner surrounding surface defines an insertion hole. The needle assembly includes a needle cannula and a needle seat securing the needle cannula. The needle seat is movable in the passage between a position of use, where a tip end of the needle cannula extends outwardly of the front open barrel end, and a disposal position, where the tip end of the needle cannula is retracted into the passage. The plunger has a front open plunger end, a rear open plunger end which extends outwardly of the rear open barrel end, and an intermediate surrounding wall which defines an accommodation chamber, and which has a rear segment proximate to the rear open plunger end. The plunger is movable in the passage between the position of use, and a retraction initiating position, where the front open plunger end is coupled with the needle seat for withdrawal of the needle seat together with the needle cannula into the accommodation chamber. An endcap has an endwall extending radially to cover the rear open plunger end, and a ring wall extending from a periphery of the endwall to surround the rear segment and to terminate at a rim that confronts the lugs. The ring wall is fittable to the inner surrounding surface so as to permit the endcap to be retained in the insertion hole once the needle seat is placed in the disposal position. The spacer has front and rear abutment surfaces which detachably and respectively abut against the peripheral edge and the rim in a preuse position to thereby guard the plunger against movement towards the retraction initiating position.

Other features and advantages of the present invention will become apparent in the following detailed description of the preferred embodiments of the invention, with reference to the accompanying drawings, in which:
Fig. 1 is a sectional view of the first preferred embodiment of a disposable syringe according to this invention;
Fig. 2 is a fragmentary sectional view of a front part of the first preferred embodiment;
Fig. 3 is a fragmentary perspective view of the first preferred embodiment, showing a tubular catch and a spacer thereof;
Fig. 4 is a sectional view of the first preferred embodiment, showing how the spacer guards against movement of a plunger;
Fig. 5 is a sectional view of the first preferred embodiment showing that the spacer is removed;
Fig. 6 is a sectional view of the first preferred embodiment in a retracted state;
Fig. 7 is a sectional view of the first preferred embodiment in a modified form;
Fig. 8 is a fragmentary sectional view of a rear part of the second preferred embodiment of a disposable syringe according to this invention;
Fig. 9 is a fragmentary perspective view of the second preferred embodiment, showing a tubular catch and a spacer thereof;
Fig. 10 is a fragmentary sectional view of a rear part of the second preferred embodiment, showing how the spacer guards against movement of a plunger;
Fig. 11 is a fragmentary sectional view of a rear part of the third preferred embodiment of a disposable syringe according to this invention;
Fig. 12 is a fragmentary perspective view of the third preferred embodiment, showing a tubular catch and a spacer thereof;
Fig. 13 is a fragmentary sectional view of a rear part of the third preferred embodiment, showing how the spacer guards against movement of a plunger;
Fig. 14 is a fragmentary sectional view of a rear part of the fourth preferred embodiment of a disposable syringe according to this invention;
Fig. 15 is a fragmentary sectional view of the fourth preferred embodiment in a retracted state;
Fig. 16 is a fragmentary sectional view of a rear part of the fifth preferred embodiment of a disposable syringe according to this invention;
Fig. 17 is a fragmentary sectional view of the fifth preferred embodiment in a retracted state;
Fig. 18 is a fragmentary sectional view of a rear part of the sixth preferred embodiment of a disposable syringe according to this invention;
Fig. 19 is a fragmentary sectional view of the sixth preferred embodiment in a retracted state; and
Fig. 20 is a sectional view of the seventh preferred embodiment of a disposable syringe according to this invention in a state of use.

Before the present invention is described in greater detail, it should be noted that same reference numerals have been used to denote like elements throughout the specification.

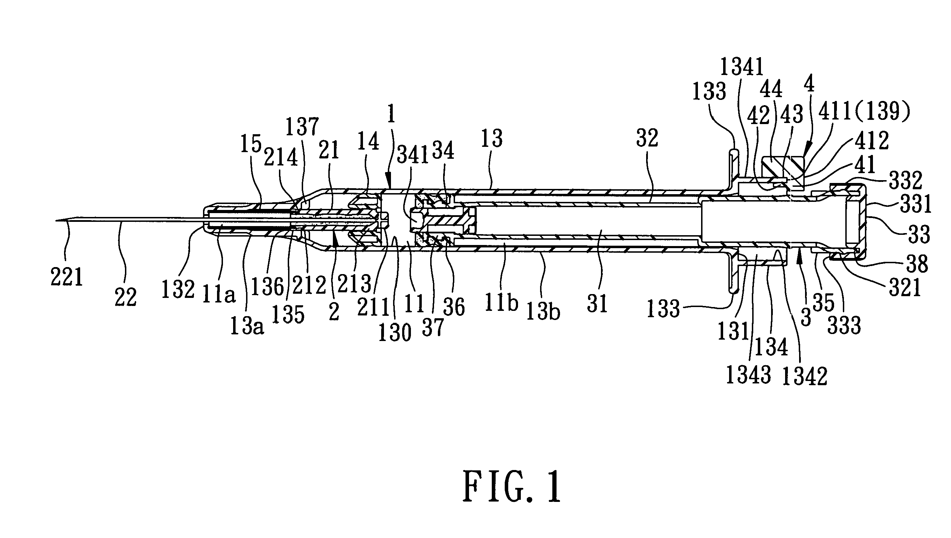

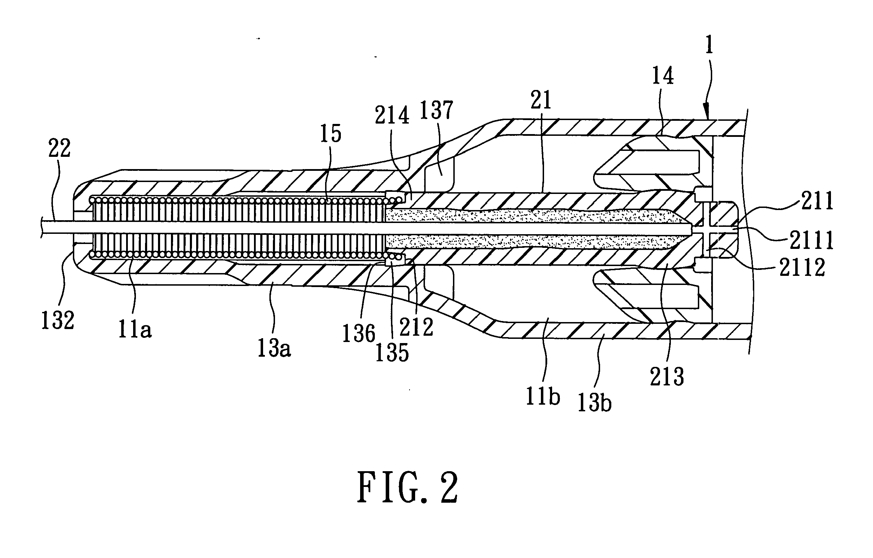

Referring to Figs. 1 to 3, the first preferred embodiment of a disposable syringe according to the present invention is shown to comprise a barrel 1, a needle assembly 2, a plunger 3, and a spacer 4.

The barrel 1 has front and rear open barrel ends 132, 131 opposite to each other along an axis, and a surrounding barrel wall 13 which interconnects the front and rear open barrel ends 132,131, and which defines a passage 11. The surrounding barrel wall 13 has small-diameter and large-diameter wall portions (13a,13b) which are disposed proximate to the front and rear open barrel ends 132,131, respectively, to divide the passage 11 into small and large passage segments (11a,11b). The small-diameter wall portion (13a) has a flange 136 confronting rearwardly, and a plurality of retaining ribs 137 disposed adjacent to the large-diameter wall portion (13b).

A pair of lugs 133 extend radially from the large-diameter wall portion (13b) and are disposed adjacent to the rear open barrel end 131. A tubular catch 134 extends from the lugs 133 along the axis and away from the surrounding barrel wall 13 to terminate at a peripheral edge 139. The tubular catch 134 has outer and inner surrounding surfaces 1341,1342 opposite to each other radially. The inner surrounding surface 1342 defines an insertion hole 1343. Preferably, the lugs 133 and the tubular catch 134 are integrally formed with the barrel 1.

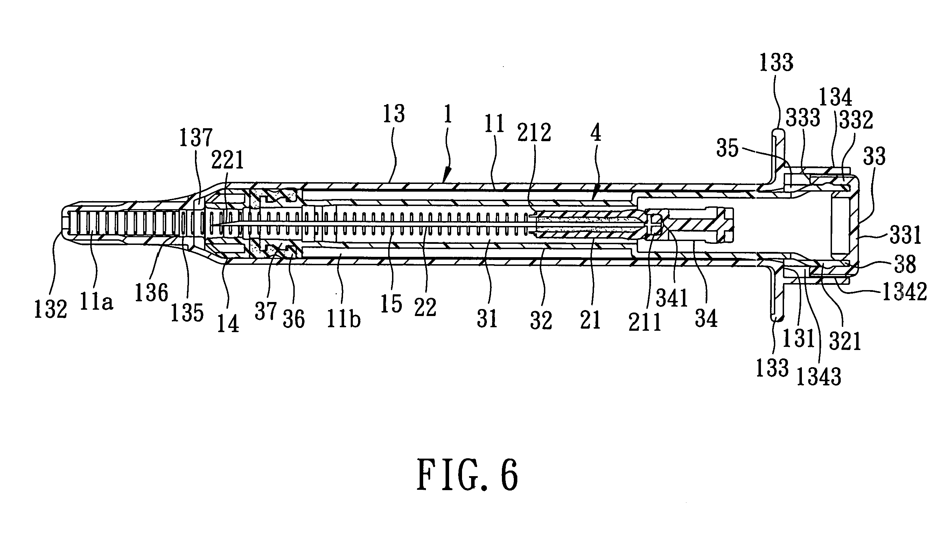

The needle assembly 2 includes a needle cannula 22 and a needle seat 21. The needle seat 21 is configured to secure the needle cannula 22, and is disposed to be movable in the passage 11 between a position of use, as shown in Fig. 1, where a tip end 221 of the needle cannula 22 extends outwardly of the front open barrel end 132, and a disposal position, as shown in Fig. 6, where the tip end 221 of the needle cannula 22 is retracted into the passage 11. The needle seat 21 has a rear seat portion 213 which is retained in the large passage segment (11b) by means of a grip member 14 so as to be placed in the position of use, and a front seat portion 214 which extends in the small passage segment (11a) and which is in frictional engagement with the small-diameter wall portion (13a) and the retaining ribs 137. The grip member 14 is in slidable air-tight engagement with the large-diameter wall portion (13b) and the rear seat portion 213, and is spaced apart from the small passage segment (11a) so as to be movable forwardly in the large passage segment (11b). The front seat portion 214 has a front abutment 212 which is spaced apart from the flange 136 along the axis in the position of use so as to define a triggering space 135 therebetween.

A coil spring 15 is disposed in the small passage segment (11a) to surround the needle cannula 22, and has front and rear biasing ends that abut against the front open barrel end 132 and the front seat portion 214, respectively, so as to be placed in a compressed state when the needle seat 21 is in the position of use.

The needle seat 21 further has an anchored portion 211 which extends rearwardly from the rear seat portion 213 along the axis. In this embodiment, the anchored portion 211 is in the form of a plug. The plug has an axial passageway 2111 and two transverse passageways 2112 which are fluidly communicated with the passage 11 and the needle cannula 22, and which respectively extend along the axis and transverse to the axis.

The plunger 3 has a front open plunger end 37 which is spaced apart from the needle seat 21, and a rear open plunger end 38 which is opposite to the front open plunger end 37 along the axis, and which extends outwardly of the rear open barrel end 131 of the barrel 1. The plunger 3 further has an intermediate surrounding wall 32 which is interposed between the front and rear open plunger ends 37,38 to define an accommodation chamber 31 therebetween, and which has a rear segment 321 proximate to the rear open plunger end 38, and a protrusion 35 proximate to the rear segment 321. A seal member 36 is retainingly sleeved on the front open plunger end 37, and is air-tightly and slidably engaged with an inner barrel surface 130 of the large-diameter wall portion (13b). A coupling rod 34 is detachably inserted into the front open plunger end 37 to close the accommodation chamber 31. The coupling rod 34 has an anchoring area 341 in the form of a socket that is configured to engage and mate with the plug of the needle seat 21, i.e., the anchored portion 211.

An endcap 33 has an endwall 331 which extends in a transverse direction relative to the axis to cover the rear open plunger end 38, and a ring wall 332 which extends from a periphery of the endwall 331 along the axis to surround the rear segment 321 and to terminate at a rim 333 that confronts the lugs 133. The ring wall 332 is configured to be fittable to the inner surrounding surface 1342 of the tubular catch 134 so as to permit the endcap 33 to be retained in the insertion hole 1343 once the needle seat 21 is placed in the disposal position, as shown in Fig. 6.

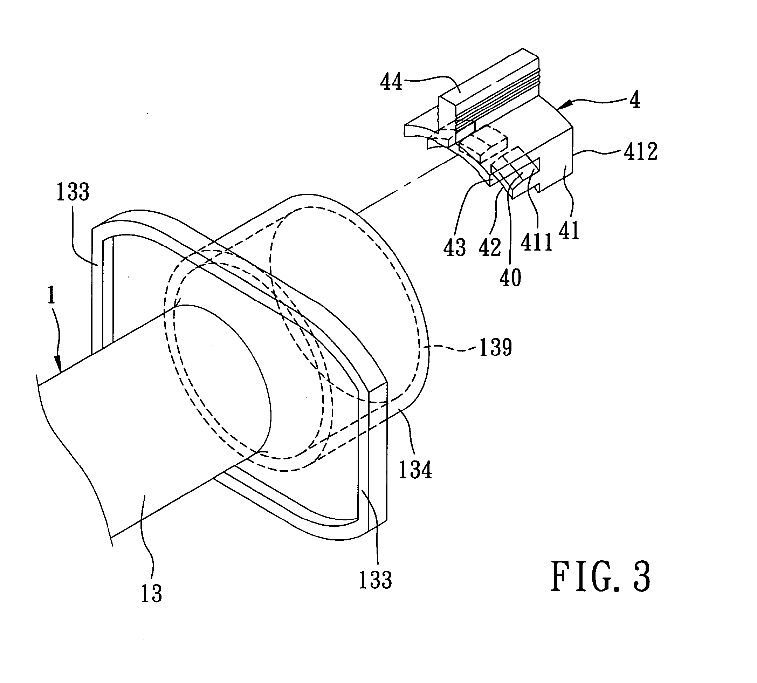

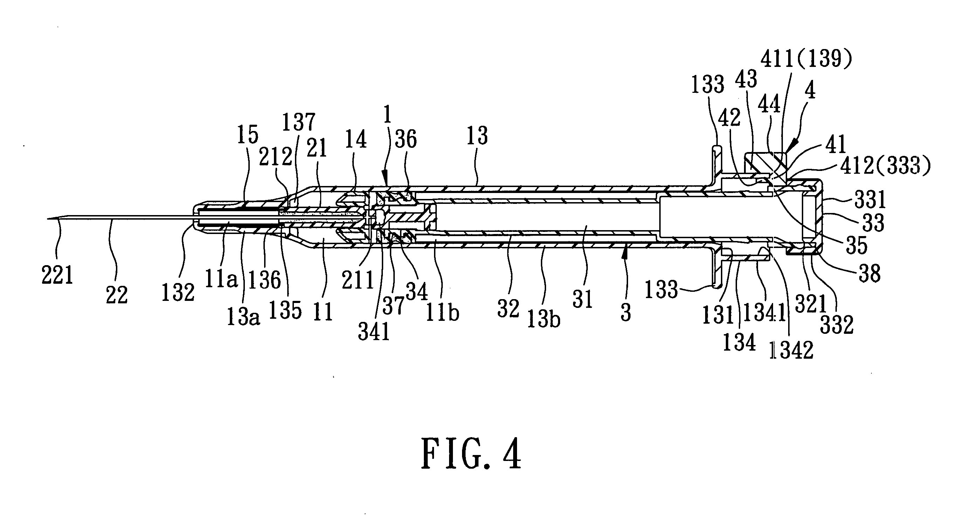

With reference to Figs. 3 and 4, the spacer 4 includes a spacer body 41 which has front and rear abutment surfaces 411,412 opposite to each other along the axis, and inner and outer gripping walls 42,43 which extend from the front abutment surface 411 along the axis, and which are spaced apart from each other to define a retaining groove 40 therebetween. The front and rear abutment surfaces 411,412 detachably and respectively abut against the peripheral edge 139 of the tubular catch 134 and the rim 333 of the endcap 33 in a preuse position to thereby prevent the plunger 3 from moving forwardly towards a retraction initiating position. The retaining groove 40 is configured to permit the tubular catch 134 to be snugly fitted therein in the pre-use position such that the inner and outer gripping walls 42,43 abut against the inner and outer surrounding surfaces 1342,1341, respectively.

The spacer 4 further has a ridge 44 which is disposed on the outer gripping wall 43 and distal from the inner gripping wall 42, and which extends along the axis to the spacer body 41 for easy manipulation.

Referring to Figs. 1, 3 and 4, before an injection operation, the user can press the plunger 3 forwards to substantially eliminate adhesion between the plunger 3 and the barrel 1 or to draw medicine liquid. As the front and rear abutment surfaces 411,412 respectively abut against the peripheral edge 139 and the rim 333, the front open plunger end 37 can be kept away from the retraction initiating position so as to prevent undesired withdrawal of the needle cannula 22 caused by inadvertent pressing of the plunger 3, thereby ensuring the effectiveness of the disposable syringe.

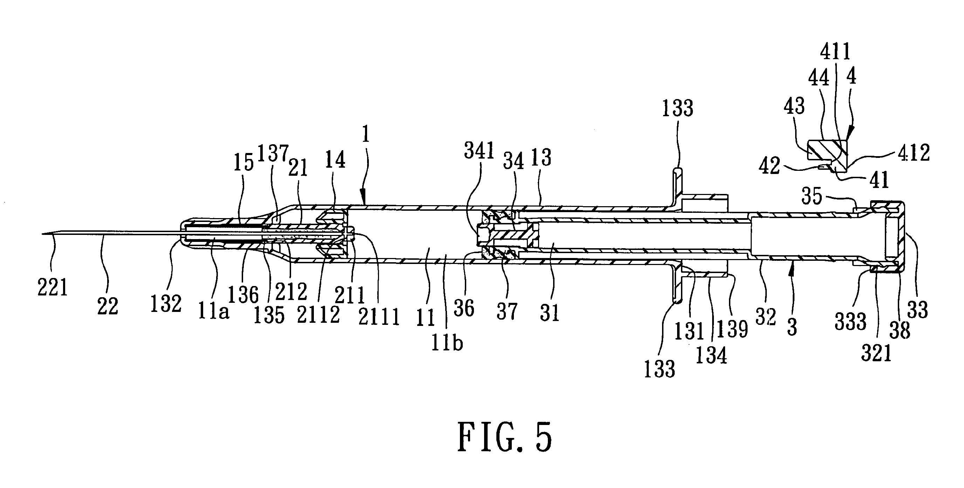

Referring to Figs. 5 and 6, during an injection operation, the user first removes the spacer 4 by pressing the ridge 44 with his/her finger. The plunger 3 is then pressed forwards until the seal member 36 abuts against the grip member 14, thereby completing the injection and placing the plunger 3 in the retraction initiating position. At this time, the anchoring area 341 of the coupling rod 34 is matingly engaged with the anchored portion 211. Hence, liquid trapped in the anchoring area 341 and the large passage segment (11b) is permitted to enter the needle cannula 22 through the axial and transverse passageways 2111,2112. Moreover, the axial and transverse passageways 2111,2112 are closed by the anchoring area 341 when the anchored portion 211 is completely inserted into and is engaged with the anchoring area 341, thereby preventing splashing of the liquid from the needle cannula 22 during a subsequent disposal operation.

When the plunger 3 is further pressed forwards to move the grip member 14 and the needle seat 21 forwardly, the front abutment 212 of the needle seat 21 is moved into the triggering space 135 to abut against the flange 136. By means of the movement of the needle seat 21 and the grip member 14, the friction and adhesion of the needle seat 21 upon the small-diameter wall portion (13a) and the friction and sticking of the grip member 14 upon the large-diameter wall portion (13b) can be diminished to facilitate subsequent withdrawal of the needle seat 21 and the needle cannula 22 into the accommodation chamber 31. At the same time, the needle seat 21 is disengaged from the grip member 14, and the coupling rod 34 is forced to move rearwards to disengage from the front open plunger end 37 so as to allow the coil spring 15 to bias the needle seat 21, as well as the needle cannula 22, to the disposal position. Thus, successful and smooth withdrawal of the needle cannula 22 is ensured.

It is noted that, after completion of needle retraction, the ring wall 332 of the endcap 33 is fitted to the inner surrounding surface 1342 of the tubular catch 134 so as to permit the endcap 33 to be retained in the insertion hole 1343, thereby preventing further manipulation of the endcap 33 for reuse. In addition, once the protrusion 35 abuts against the rear open barrel end 131, further forward movement of the plunger 3 is checked so as to prevent application of excess compression force to the grip member 14, which may result in deformation of the grip member 14 and may therefore hinder retraction of the needle seat 21.

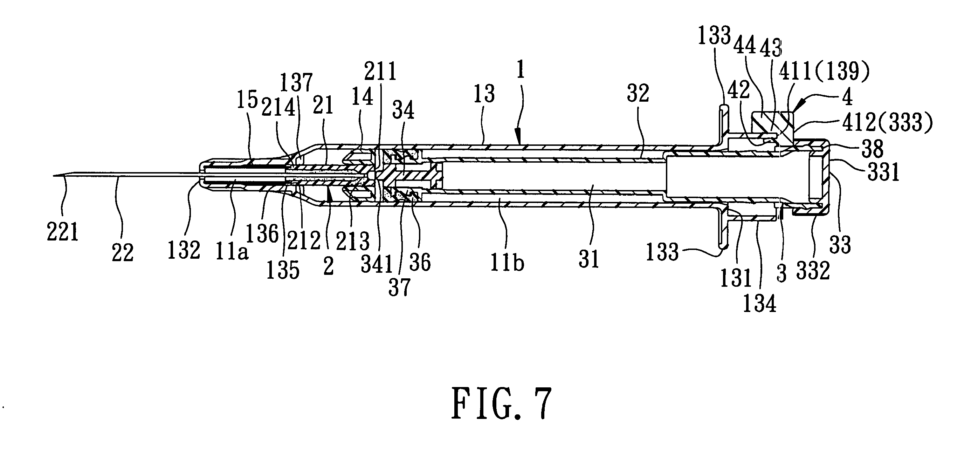

Referring to Fig. 7, alternatively, the anchored portion 211 and the anchoring area 341 are in the form of a socket and a plug, respectively, which can engage and mate with each other for withdrawal of the needle seat 21 together with the needle cannula 22 into the accommodation chamber 31.

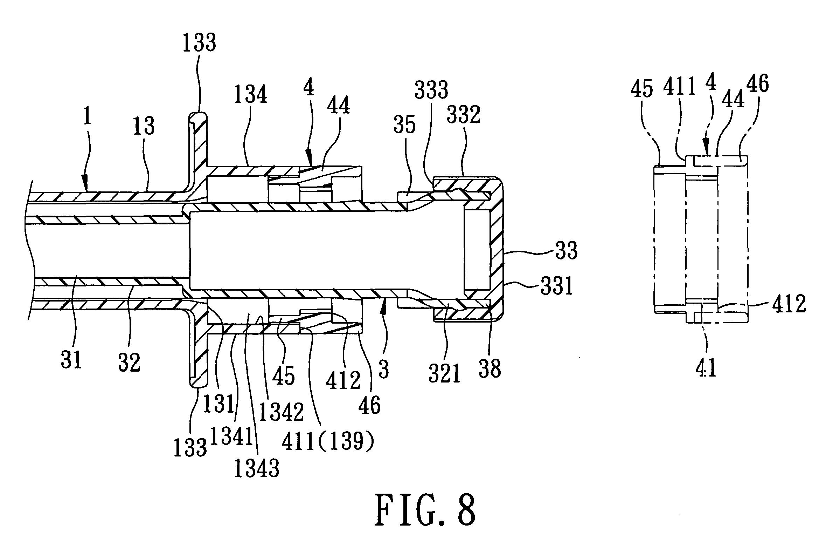

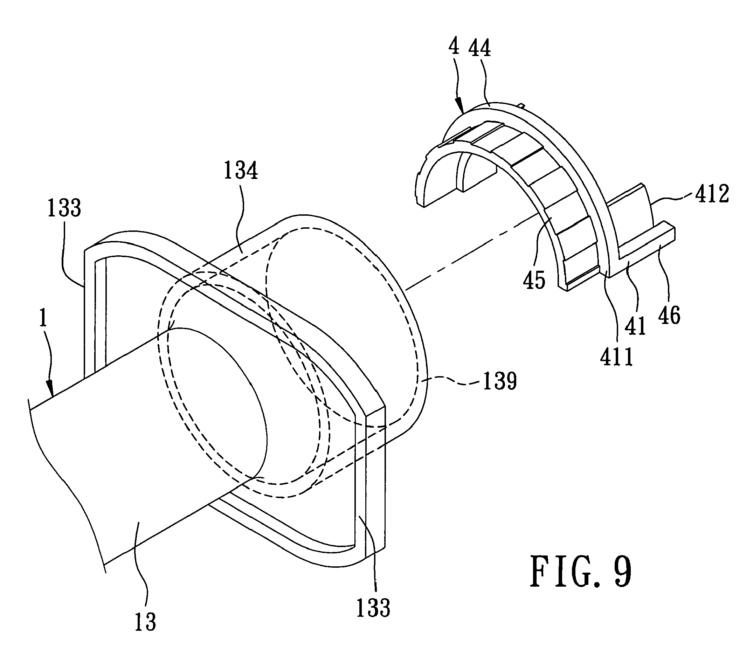

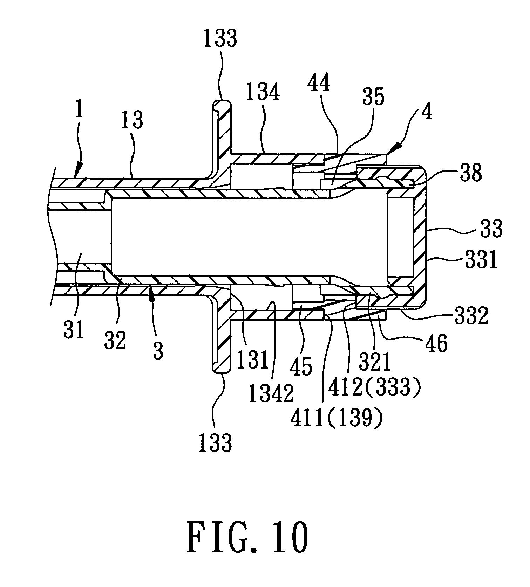

Referring to Figs. 8 to 10, the second preferred embodiment of a disposable syringe according to this invention is similar to the first preferred embodiment in construction, except that the spacer 4 further includes head and leg portions 45,46 which are disposed forwardly of the front abutment surface 411 and rearwardly of the rear abutment surface 412, respectively, and which are offset from each other radially such that the head and leg portions 45,46 respectively serve as power and weight ends of a lever for acquiring a propensity to abut against the inner surrounding surface 1342 of the tubular catch 134 and the ring wall 332 of the endcap 33, respectively, so as to thereby help stabilize the spacer body 41 in the pre-use position. The head portion 45 is configured to extend angularly about the axis, such as having a semi-circular shape, and is formed with a friction surface such that firm engagement of the head portion 45 with the inner surrounding surface 1342 of the tubular catch 134 can be ensured by virtue of an increased frictional force generated therebetween.

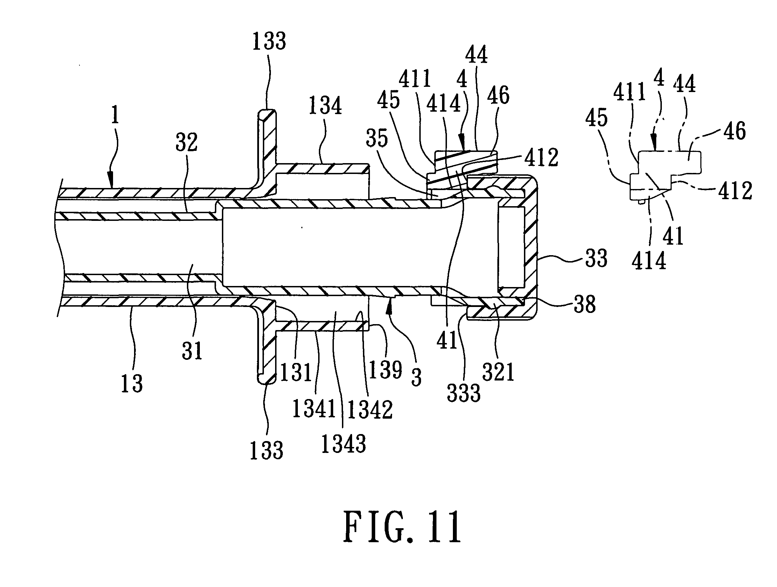

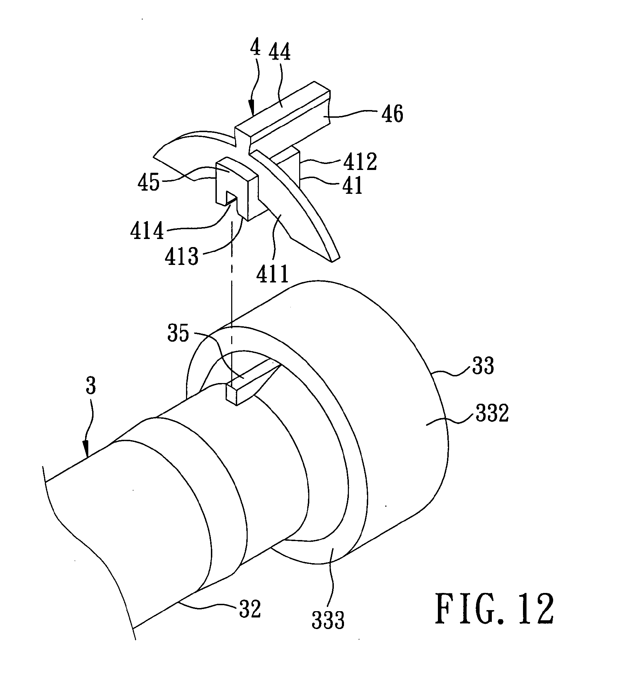

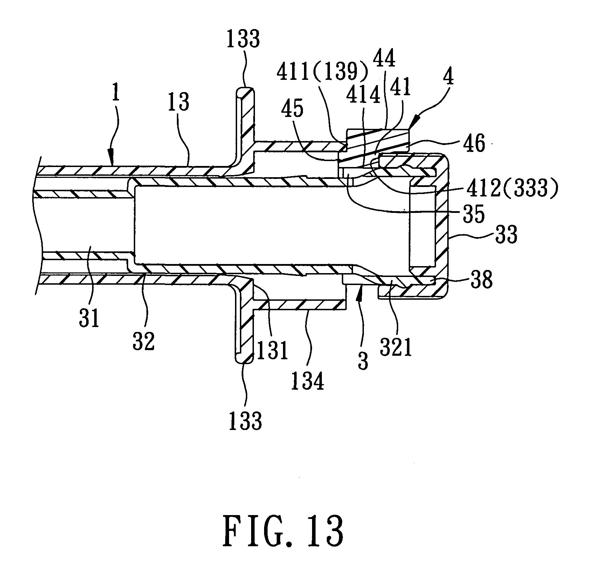

Referring to Figs. 11 to 13, the third preferred embodiment of a disposable syringe according to this invention is similar to the first preferred embodiment in construction, except that the spacer body 41 of the spacer 4 includes a mounting surface 413 which extends along the axis, and which confronts the intermediate surrounding wall 32 of the plunger 3 radially. In addition, there is provided a mortise joint that includes the protrusion 35 which is in the form of a tenon 35 and which is disposed on the intermediate surrounding wall 32 of the plunger 3 adjacent to the rear segment 321, and a mortise 414 which is disposed in the mounting surface 413 for receiving the tenon 35 fittingly so as to prevent the spacer 4 from moving angularly about the axis in the preuse position.

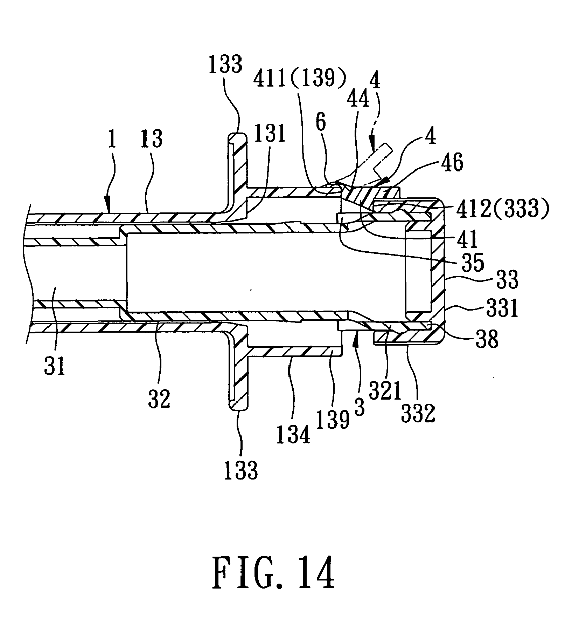

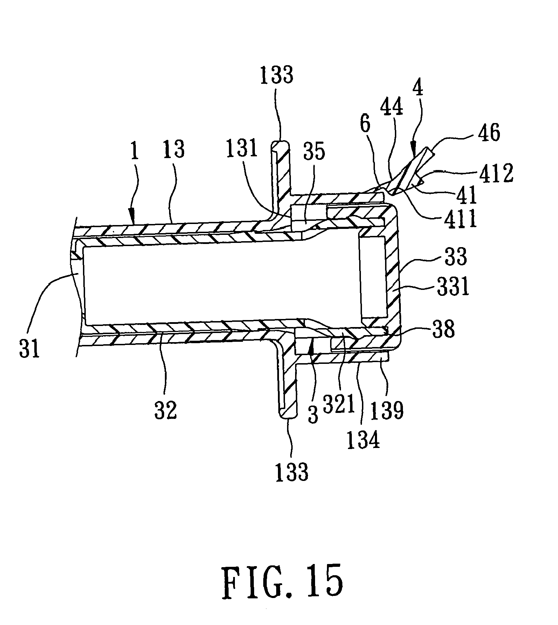

Referring to Figs. 14 and 15, the fourth preferred embodiment of a disposable syringe according to this invention is similar to the first preferred embodiment in construction, except that a flexible connecting strap 6 is further disposed to interconnect the tubular catch 134 and the spacer 4, and is configured such that the front and rear abutment surfaces 411,412 are permitted to be removed from the peripheral edge 139 of the tubular catch 134 and the rim 333 of the endcap 33. In this embodiment, the flexible connecting strap 6 is integrally formed with the spacer 4 and is disposed adjacent to the front abutment surface 411 to engage the peripheral edge 139 of the tubular catch 134. In addition, the spacer 4 further has a leg portion 46 which is disposed rearwardly of the rear abutment surface 412 to abut against the ring wall 332 of the endcap 33 so as to help stabilize the spacer body 41 in the pre-use position.

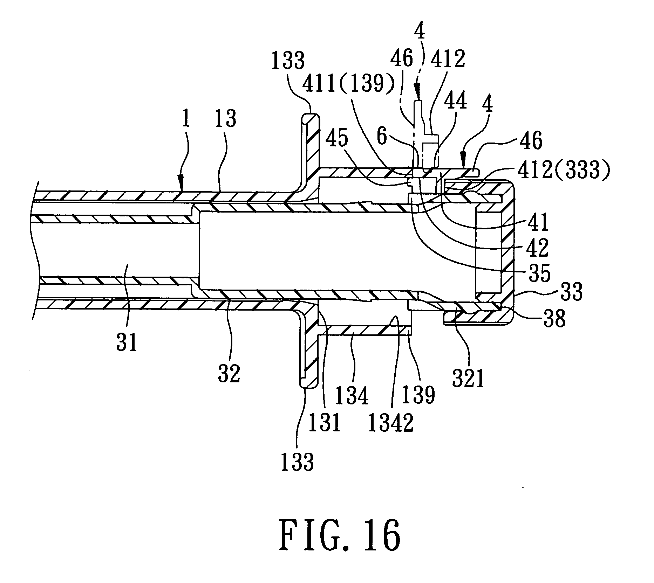

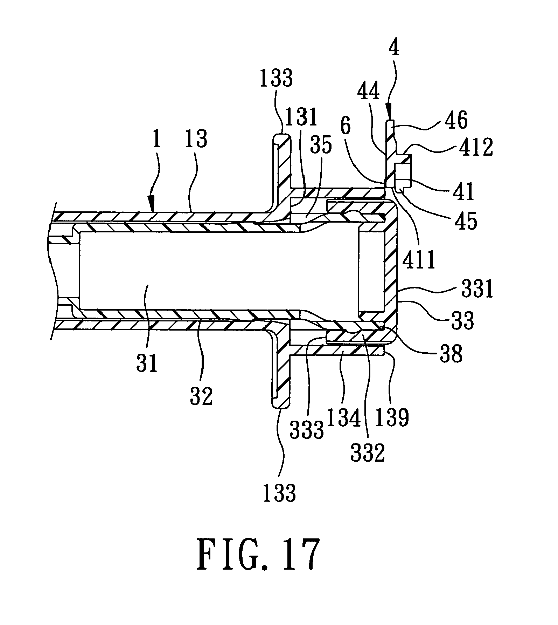

Referring to Figs. 16 and 17, the fifth preferred embodiment of a disposable syringe according to this invention is similar to the fourth preferred embodiment in construction, except that the spacer 4 further has a head portion 45 which is disposed forwardly of the front abutment surface 411 to abut against the inner surrounding surface 1342 of the tubular catch 134.

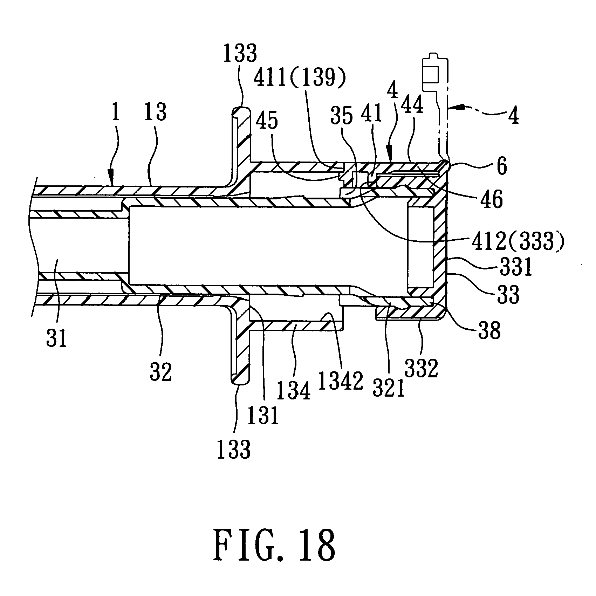

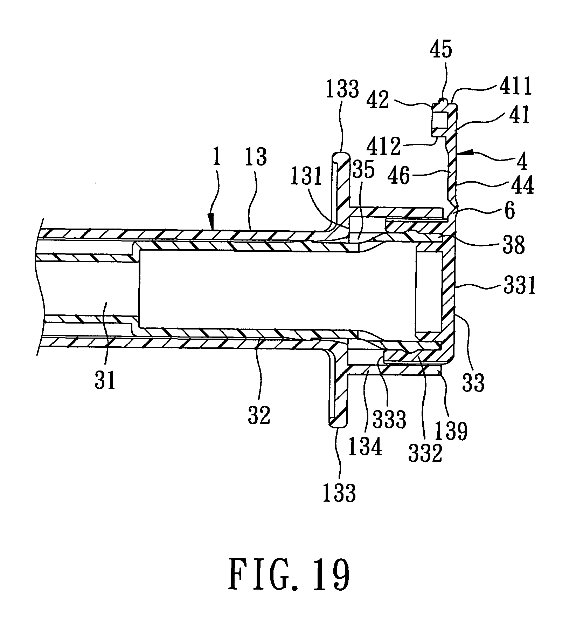

Referring to Figs. 18 and 19, the sixth preferred embodiment of a disposable syringe according to this invention is similar to the fourth preferred embodiment in construction, except that the flexible connecting strap 6 is disposed to interconnect the leg portion 46 of the spacer 4 and the periphery of the endwall 331 of the endcap 33. In addition, the spacer 4 further has a head portion 45 which is disposed forwardly of the front abutment surface 411 to abut against the inner surrounding surface 1342 of the tubular catch 134 so as to help stabilize the spacer body 41 in the preuse position.

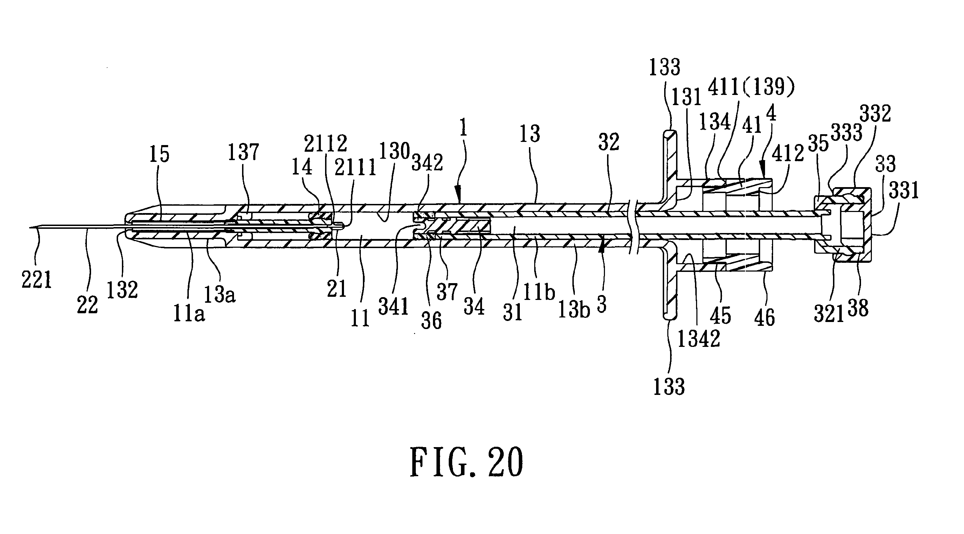

In the aforesaid embodiments, a disposable syringe is provided for injection of medication of a general volume, such as 3 ml, 5 ml or more. In the seventh preferred embodiment of this invention, as shown in Fig. 20, the disposable syringe is used for injection of medication of a very small volume, such as 1 ml or 0.5 ml. In other words, the barrel 1, the needle assembly 2, the plunger 3, and the spacer 4 have a relatively small structure.

In order to obtain a slenderized construction of a disposable syringe of the present invention, the coupling rod 34 has a front coupling portion 342 which extends forwardly beyond the front open plunger end 37 and which has an anchoring area 341 in the form of a socket. The seal member 36 is retainingly sleeved on the front coupling portion 342, and is air-tightly and slidably engaged with the inner barrel surface 130 of the large-diameter wall portion (13b).

It is noted that, since the needle seat 21 is movably engaged with the grip member 14 and is largely disposed in the large passage segment (11b) of the barrel 1, and since the coil spring 15 of this invention is disposed to surround the needle cannula 22 with the rear biasing end abutting against the front seat portion 214 of the needle seat 21, the diameter of the coil spring 15 and the diameter of the needle seat 21 can be relatively small. Thus, the inner diameter of the barrel 1 for accommodating the plunger 3, which in turn accommodates the retracted needle seat 21, can be made comparatively small so as to slenderize the whole construction of the disposable syringe for convenient detailed calibration of small volumes, such as 0.5 ml and 1 ml. In addition, compared with the syringes of the previous embodiments in which the seal member 36 is sleeved on the front open plunger end 37 of the plunger 3, the seal member 36 of this embodiment is sleeved on the front coupling portion 342 of the coupling rod 34, thereby maximizing the inner diameter of the front open plunger end 37 for facilitating entry of the retracted needle seat 21 into the accommodation chamber 31 of the comparatively slender plunger 3.

## Claims

1. A disposable syringe guarded in a preuse position, comprising:
a barrel (1) having front and rear open barrel ends (132,131) opposite to each other along an axis, and a surrounding barrel wall (13) which interconnects said front and rear open barrel ends (132,131), and which defines a passage (11);
a pair of lugs (133) which extend radially from said surrounding barrel wall (13) and which are disposed adjacent to said rear open barrel end (131);
a needle cannula (22);
a needle seat (21) which is configured to secure said needle cannula (22), and which is disposed to be movable in said passage (11) between a position of use, where a tip end (221) of said needle cannula (22) extends outwardly of said front open barrel end (132), and a disposal position, where said tip end (221) of said needle cannula (22) is retracted into said passage (11);
a plunger (3) having a front open plunger end (37) which is spaced apart from said needle seat (21), and a rear open plunger end (38) which is opposite to said front open plunger end (37) along the axis, and which extends outwardly of said rear open barrel end (131), said plunger (3) further having an intermediate surrounding wall (32) which is interposed between said front and rear open plunger ends (37,38), which defines an accommodation chamber (31), and which has a rear segment (321) proximate to said rear open plunger end (38), said plunger (3) being movable in said passage (11) between the position of use and a retraction initiating position, where said front open plunger end (37) is coupled with said needle seat (21) for withdrawal of said needle seat (21) together with said needle cannula (22) into said accommodation chamber (31), **characterized by:**
a tubular catch (134) which extends from said lugs (133) along the axis and away from said surrounding barrel wall (13) to terminate at a peripheral edge (139), and which has outer and inner surrounding surfaces (1341,1342) opposite to each other radially, said inner surrounding surface (1342) defining an insertion hole (1343);
an endcap (33) which has an endwall (331) having a periphery and extending in a transverse direction relative to the axis to cover said rear open plunger end (38), and a ring wall (332) extending from said periphery of said endwall (331) along the axis to surround said rear segment (321) and to terminate at a rim (333) that confronts said lugs (133), said ring wall (332) being configured to be fittable to said inner surrounding surface (1342) so as to permit said endcap (33) to be retained in said insertion hole (1343) once said needle seat (21) is placed in the disposal position; and
a spacer (4) having front and rear abutment surfaces (411, 412) which are opposite to each other along the axis, and which detachably abut against said peripheral edge (139) and said rim (333), respectively, in a pre-use position to thereby prevent said plunger (3) from moving towards the retraction initiating position.

2. The disposable syringe according to Claim 1, **characterized in that** said spacer (4) includes a spacer body (41) which has said front and rear abutment surfaces (411,412), and inner and outer gripping walls (42,43) which extend from said front abutment surface (411) along the axis, and which are spaced apart from each other to define a retaining groove (40), said retaining groove (40) being configured to permit said tubular catch (134) to be snugly fitted therein in the pre-use position such that said inner and outer gripping walls (42,43) abut against said inner and outer surrounding surfaces (1342,1341), respectively.

3. The disposable syringe according to Claim 2, **characterized in that** said spacer (4) has a ridge (44) which is disposed on said outer gripping wall (43) and distal from said inner gripping wall (42), and which extends along the axis to said spacer body (41) for easy manipulation.

4. The disposable syringe according to Claim 1, **characterized in that** said spacer (4) includes a spacer body (41) which has said front and rear abutment surfaces (411,412), and head and leg portions (45,46) which are disposed forwardly of said front abutment surface (411) and rearwardly of said rear abutment surface (412), respectively, and which are offset from each other radially such that said head and leg portions (45,46) acquire a propensity to abut against said inner surrounding surface (1342) of said tubular catch (134) and said ring wall (332) of said endcap (33), respectively, to thereby help stabilize said spacer body (41) in the pre-use position.

5. The disposable syringe according to Claim 4, **characterized in that** said head portion (45) is configured to extend angularly about the axis such that firm engagement of said head portion (45) with said inner surrounding surface (1342) of said tubular catch (134) is ensured by virtue of an increased frictional force generated therebetween.

6. The disposable syringe according to Claim 1, **characterized in that** said spacer (4) includes a spacer body (41) which has said front and rear abutment surfaces (411,412) and which includes a mounting surface (413) that extends along the axis, and that confronts said intermediate surrounding wall (32) of said plunger (3) radially, said disposable syringe further comprising a mortise joint which has a tenon (35) disposed on said intermediate surrounding wall (32) adjacent to said rear segment (321), and a mortise (414) disposed in said mounting surface (413) for receiving said tenon (35) fittingly so as to prevent said spacer (4) from moving angularly about the axis.

7. The disposable syringe according to Claim 6, **characterized in that** said spacer (4) includes a ridge (44) disposed on said spacer body (41) opposite to said mounting surface (413) radially for easy manipulation.

8. The disposable syringe according to Claim 1, **further characterized by** a flexible connecting strap (6) which interconnects said tubular catch (134) and said spacer (4) and which is configured such that said front and rear abutment surfaces (411,412) are permitted to be removed from said peripheral edge (139) and said rim (333).

9. The disposable syringe according to Claim 1, **further characterized by** a flexible connecting strap (6) which interconnects said endcap (33) and said spacer (4) and which is configured such that said front and rear abutment surfaces (411,412) are permitted to be removed from said peripheral edge (139) and said rim (333).

10. The disposable syringe according to Claim 1, **further characterized by** a coil spring (15) which is disposed between said needle seat (21) and said front open barrel end (132) and which surrounds said needle cannula (22) to be placed in a compressed state when said needle seat (21) is in the position of use.

11. The disposable syringe according to Claim 10, **characterized in that** said surrounding barrel wall (13) has small-diameter and large-diameter wall portions (13a,13b) which are disposed proximate to said front and rear open barrel ends (132,131), respectively, to divide said passage (11) into small and large passage segments (11a,11b), said needle seat (21) having a rear seat portion (213) which is retained in said large passage segment (13b) so as to be placed in the position of use, and a front seat portion (214) which extends in said small passage segment (11a) and which is in frictional engagement with said small-diameter wall portion (13a), said coil spring (15) having front and rear biasing ends which abut against said front open barrel end (132) and said front seat portion (214), respectively.

12. The disposable syringe according to Claim 11, **characterized in that** said small-diameter wall portion (13a) has a flange (136) which confronts rearwardly, said front seat portion (214) having a front abutment (212) which is spaced apart from said flange (136) along the axis in the position of use so as to define a triggering space (135) therebetween, such that, by virtue of forward movement of said plunger (3) to couple said front open plunger end (37) with said needle seat (21), said front abutment (212) is moved into said triggering space (135) to abut against said flange (136).

13. The disposable syringe according to Claim 11, **further characterized by** a grip member (14) which is in slidable and air-tight engagement with said large-diameter wall portion (13b) and said rear seat portion (213) and which is spaced apart from said small passage segment (11a) so as to be movable forwardly in said large passage segment (11b).

14. The disposable syringe according to Claim 11, **characterized in that** said needle seat (21) has an anchored portion (211) which extends rearwardly from said rear seat portion along the axis, said disposable syringe further comprising a coupling rod (34) which is detachably inserted into said front open plunger end (37) to close said accommodation chamber (31), and which has an anchoring area (341) that is engageable with said anchored portion (211) so as to couple said plunger (3) with said needle seat (21) for withdrawal of said needle seat (21).

15. The disposable syringe according to Claim 14, **characterized in that** said anchored portion (211) and said anchoring area (341) are, respectively, a plug and a socket which engage and mate with each other, said plug having axial and transverse passageways (2111,2112) which are fluidly communicated with said passage (11) and said needle cannula (22), and which respectively extend along the axis and transverse to the axis, such that liquid trapped in said socket and said passage (11) is permitted to enter said needle cannula (22) through said axial and transverse passageways (2111,2112), respectively, when said plug is inserted into said socket, and such that said axial and transverse passageways (2111,2112) are closed by said socket when said plug is completely inserted into and is engaged with said socket so as to prevent splashing of the liquid from said needle cannula (22).

16. The disposable syringe according to Claim 15, **characterized in that** said coupling rod (34) has a front coupling portion (342) which extends forwardly beyond said front open plunger end (37) and which defines said socket therein, said disposable syringe further comprising a seal member (36) which is retainingly sleeved on said front coupling portion (342) and which is air-tightly and slidably engaged with an inner barrel surface (130) of said large-diameter wall portion (13b).

17. The disposable syringe according to Claim 14, **characterized in that** said anchored portion (211) and said anchoring area (341) are, respectively, a socket and a plug which engage and mate with each other.

18. The disposable syringe according to Claim 14, **further characterized by** a seal member (36) which is retainingly sleeved on said front open plunger end (37) and which is air-tightly and slidably engaged with an inner barrel surface (130) of said large-diameter wall portion (13b).

19. The disposable syringe according to Claim 1, **characterized in that** said intermediate surrounding wall (32) of said plunger (3) has a protrusion (35) which is disposed to abut against said rear open barrel end (131) to prevent excess forward movement of said plunger (3).
